# EUROPÄISCHE PATENTSCHRIFT

(11) **EP 0 697 416 B1**
(45) Veröffentlichungstag und Bekanntmachung des Hinweises auf die Patenterteilung: **28.01.1998**
(21) Anmeldenummer: 95112275.3
(22) Anmeldetag: 04.08.1995
(51) Int. Cl.: C08F 8/12, C08F 8/44

(54) **Superabsorbierende Polymere aus vernetzten Polyacrylnitril-Emulsionen**
Superabsorbent polymers from crosslinked poly(acrylonitrile) emulsions
Polymères superabsorbants d'emulsions de poly(acrylonitrile) réticulées

(30) Priorität: 18.08.1994 DE 4429318
(43) Veröffentlichungstag der Anmeldung: 21.02.1996
(73) Patentinhaber: BAYER AG, 51368 Leverkusen (DE)
(72) Erfinder: Sackmann, Günter, Dr., D-51379 Leverkusen (DE); Korte, Siegfried, Dr., D-51519 Odenthal (DE); Schapowalow, Sergei, Dr., D-51373 Leverkusen (DE); Szablikowski, Klaus, Dr., D-29664 Walsrode (DE); Koch, Wolfgang, Dr., D-29699 Bomlitz (DE)

(56) Entgegenhaltungen:
- EP-A- 0 047 381
- EP-A- 0 170 081
- EP-A- 0 241 885
- EP-A- 0 406 648
- EP-A- 0 670 335
- GB-A- 1 104 567
- US-A- 3 929 740

## Beschreibung

Die Erfindung betrifft die Herstellung von superabsorbierenden Polymerisaten in Pulverform mit extrem hohem Quellungsvermögen und ausgezeichneten Gelfestigkeiten.

Superabsorbierende Polymerisate sind bekannt und werden hauptsächlich bei der Herstellung von Windeln und Inkontinenzartikeln, aber auch als wasserspeichernde Materialien in der Landwirtschaft sowie bei der Ummantelung von Elektrokabeln verwendet. In der Regel sind diese superabsorbierenden Polymerisate weitmaschig vernetzte, wasserunlösliche Polymerisate auf Basis von Alkali salzen der Polyacrylsäure oder Copolymerisate von Alkalisalzen der Acrylsäure und Acrylamid, die durch radikalisch initiierte Copolymerisation von Acrylsäure und polyfunktionellen Monomeren, wie Divinylbenzol, Ethylenglykoldimethacrylat, Ethylenglykoldiallylether, Butandiolacrylat, Hexandiolmethacrylat, Polyglykoldiacrylat, Trimethylolpropandiacrylat, Trimethylolpropantrisacrylat, Allylacrylat, Diallylacrylamid, Triallylamin, Diallylether, Methylenbisacrylamid und N-Methylolacrylamid, erhalten werden. Aufgrund ihrer Struktur sind solche Polymerisate in der Lage, unter Quellung und Ausbildung von Hydrogelen große Mengen an Flüssigkeiten aufzunehmen und diese auch unter Druck festzuhalten.

Es gibt auch superabsorbierende Polymerisate, die auf Hydrolysaten von Pfropfcopolymerisaten des Acrylnitrils auf Stärke sowie auf vernetzten Stärke-Acrylsäure-Pfropfcopolymerisaten, bei denen die Carboxylgruppen teilweise neutralisiert sind, basieren.

Auch die saure oder alkalische Hydrolyse von Polyacrylnitril führt zu Polymeren mit Carboxyl- bzw. Carboxylatgruppen. Diese sind in der Regel wasserlöslich (und daher nicht quellbar), denn das als Ausgangsprodukt eingesetzte und durch Fällungspolymerisation gewonnene Polyacrylnitril hat ein zu niedriges Molekulargewicht.

Erfindungsgemäß können aus vernetzten wäßrigen Acrylnitrilpolymerisat-Emulsionen superabsorbierende Polymerisate mit hohem Quellungs-(Wasserrückhalte)vermögen und ausgezeichneter Gelfestigkeit hergestellt werden.

Gegenstand der Erfindung ist somit ein Verfahren zur Herstellung von vernetzten superabsorbierenden Polymerisaten mit einem Quellungsvermögen für wäßrige Flüssigkeiten bis zu 700 g/g und für Elektrolytlösungen bis zu 60 g/g sowie Gelfestigkeiten von 20 bis 100 g, worin man wäßrige Emulsionen von schwach bis mäßig vernetzten Homo- und/oder Copolymerisaten des Acrylnitrils durch Umsetzung mit wäßrigen Lösungen von Alkalihydroxiden partiell hydrolysiert, die Polymerisate durch Zugabe von mit Wasser mischbaren organischen Lösungsmitteln als Pulver ausfällt, abtrennt, trocknet und dann gegebenenfalls für eine kurze Zeit erhitzt.

Wie aus der Deutschen Patentanmeldung P 42 33 026.2 bekannt ist, können mit Hilfe spezieller anionischer polymerer Emulgatoren hochkonzentrierte wäßrige Emulsionen von Homo- und Copolymerisaten des Acrylnitrils mit mittleren Teilchendurchmessern von 100 bis 300 nm hergestellt werden. Setzt man nun bei der Herstellung dieser Emulsionen ein Gemisch aus 96,0 bis 99,8 Gew.-% Acrylnitril und 4,0 bis 0,2 Gew.-% eines polyfunktionellen Monomeren ein, dann erhält man ebenfalls feinteilige Emulsionen mit Teilchengrößen zwischen 100 und 300 nm. Die dabei gebildeten Acrylnitril-Polymerisate sind vernetzt und können nach Ausfällen aus der Emulsion nicht mehr in den für Polyacrylnitril geeigneten Lösungsmitteln, wie z. B. DMF oder DMAc gelöst werden. Werden diese wäßrigen Acrylnitrilpolymerisat-Emulsionen mit wäßrigen Lösungen von Alkalihydroxiden behandelt, dann findet bei 50 bis 100°C, bevorzugt bei 70 bis 95°C, innerhalb kurzer Zeit eine partielle Umwandlung der Nitrilgruppen in Carboxylatund Carbonamidgruppen statt. Nach Aufarbeitung erhält man vernetzte, unlösliche, in Wasser und Elektrolytlösungen sowie Blut stark quellende Pulver mit äußerst günstigen superabsorbierenden Eigenschaften.

Ein weiterer Gegenstand der Erfindung sind superabsorbierende Polymerisate auf Basis teilhydrolysierter, durch den Einbau polyfunktioneller Monomerer vernetzter Homo- und/oder Copolymerisate des Acrylnitrils, worin 30 bis 80 Mol-% der Nitrilgruppen in Carboxylatgruppen, 20 bis 70 Mol-% der Nitrilgruppen in Carbonamidgruppen und 0 bis 20 Mol-% der Nitrilgruppen unverändert erhalten sind, wobei die vernetzten Polymerisate ein Quellungsvermögen von bis zu 700 g/g in Wasser und bis zu 60 g/g in physiologischer Kochsalzlösung sowie Gelfestigkeiten von 20 bis 100 g besitzen.

Bevorzugt sind solche superabsorbierenden Polymerisate, in denen 30 bis 50 Mol-% der Nitrilgruppen in Carboxylatgruppen und 20 bis 40 Mol-% der Nitrilgruppen in Carbonamidgruppen umgewandelt sind, während 5 bis 20 Mol-% unverändert vorliegen.

Die Herstellung von wäßrigen Emulsionen von unvernetzten hochmolekularen Homo- oder Copolymerisaten des Acrylnitrils ist in der Deutschen Patentschrift P 42 33 026.2 beschrieben. Nach diesem Verfahren, das in Gegenwart eines polymeren anionischen Emulgators durchgeführt wird, und zu feinteiligen (mittlerer Teilchendurchmesser: 100 bis 300 nm, ermittelt durch Laser-Korrelations-Spektroskopie) Polymerisatemulsionen mit sehr hohen Molekulargewichten führt, können die Ausgangsmaterialien zur Herstellung der erfindungsgemäßen superabsorbierenden Polymerisate gewonnen werden. Ihre mittleren Molekulargewichte (Gewichtsmittel, ermittelt durch Gelpermeationschromatographie) sind 5•10⁵ g/Mol bis 1•10⁷ g/Mol, bevorzugt 2•10⁶ g/Mol bis 5•10⁶ g/Mol.

Nach demselben Verfahren lassen sich ebenfalls vernetzte, unlösliche Homo- oder Copolymerisate mit Teilchengrößen zwischen 100 und 300 nm herstellen, wenn man dem oder den Ausgangsmonomeren zwischen 0,2 und 4,0 Gew.-% an polyfunktionellen Monomeren, wie z. B. Divinylbenzol, Ethylenglykoldimethacrylat, Ethylenglykoldiallylether, Butandiolacrylat, Hexandiolmethacrylat, Polyethylenglykoldimethacrylat, Trimethylolpropantrisacrylat, Allylacrylat, Diallyl acrylamid, Triallylamin, Diallylether oder Methylenbisacrylamid, zumischt. Nach Ausfällen aus der Emulsion sind diese Produkte in für die Lösung von Polyacrylnitril geeigneten Lösungsmitteln, wie z. B. DMF oder DMAc, unlöslich. Der Vernetzungsgrad der auf diese Weise erhaltenen Polymerisate richtet sich nach der Menge an eingesetztem polyfunktionellem Monomerem und kann sich von sehr weitmaschig bis sehr engmaschig erstrecken.

Die beispielweise nach diesem Verfahren erhaltenen Polymerisatdispersionen können direkt mit den Alkalihydroxiden umgesetzt werden; aufgrund der kleinen Teilchengrößen und somit der großen Oberfläche der Polymerisatteilchen sind sehr kurze Reaktionszeiten möglich.

Zur Umsetzung (Hydrolyse) kann man wäßrige, bevorzugt 0,5 bis 10 gew.-%ige Lösungen von Alkalihydroxiden, z. B. NaOH oder KOH verwenden. Das molare Verhältnis von Nitrilgruppen der Ausgangspolymerisate zu den Hydroxylgruppen der Alkalihydroxide ist bevorzugt 1:0,9 bis 1:0,1, besonders bevorzugt 1:0,7 bis 1:0,3. Produkte mit besonders günstigen anwendungstechnischen Eigenschaften werden erhalten, wenn dieses Verhältnis bei 1:0,5 bis 1:0,4 liegt. Die Hydrolyse wird in der Regel bei 50 bis 100°C, bevorzugt 70 bis 95°C durchgeführt. Der Neutralisationsgrad der bei der Hydrolysereaktion entstehenden Carboxylgruppen kann durch Zugabe von entsprechenden Mengen an Mineralsäuren, wie z. B. HCl, verändert werden. So sind meist zwischen 30 und 100 %, bevorzugt zwischen 50 und 70 %, der Carboxylgruppen im Endprodukt neutralisiert, während die restlichen Carboxylgruppen in der Säureform vorliegen.

Die erforderliche Reaktionszeit hängt von der Reaktionstemperatur und dem gewünschten Hydrolysegrad ab (dieser ist natürlich auch eine Funktion der Reaktionstemperatur). In der Regel werden 20 bis 90 Mol-% der Acrylnitrilreste in den vernetzten Acrylnitril-Homo- oder ∼-Copolymerisaten in Carboxylat- bzw. Carbonamidgruppen umgewandelt. Durch den Hydrolysegrad können die Eigenschaften der vernetzten superabsorbierenden Polymerisate beeinflusst werden: so steigt für einen bestimmten Vernetzungsgrad der maximal erreichbare Quellungsgrad mit dem Gehalt an Carboxylatgruppen an. Doch entscheidend für die Qualität der vernetzten superabsorbierenden Polymerisate ist die Gelfestigkeit der gequollenen Pulver, welche durch den Vernetzungsgrad bestimmt wird. Mit zunehmendem Vernetzungsgrad nimmt die Gelfestigkeit der Produkte zu, während der maximale Quellungsgrad abnimmt. Um also Produkte mit einem für einen bestimmten Einsatzbereich optimalen Eigenschaftsprofil zu erhalten, muß durch die Menge an polyfunktionellem Monomerem im Ausgangsmonomerengemisch ein bestimmter Vernetzungsgrad der Polyacrylnitril-Emulsion eingestellt werden und bei der anschließenden Hydrolyse empirisch die Reaktionstemperatur, die Reaktionszeit und das Molverhältnis Nitrilgruppen zu Hydroxylgruppen so gewählt werden, daß der gewünschte Hydrolysegrad erhalten wird.

Zur Isolierung der vernetzten superabsorbierenden Polymerisate in Pulverform gibt man nach Beendigung der Hydrolyse unter starkem Rühren mit Wasser mischbare organische Lösungsmittel, wie z. B. Aceton, Methanol oder Ethanol, zu, in einer Menge, die bis zum 3-fachen Volumen des wäßrigen Reaktionsgemischs betragen kann. Bevorzugt wird Ethanol in einem Volumenverhältnis 1:1 eingesetzt. Dabei fallen die superabsorbierenden Polymerisate als feinteilige, leicht filtrierbare Pulver aus. Nach ihrer Trocknung bei 50 bis 100°C können sie durch Sieben nach ihren Korngrößen klassiert werden. Die Korngrößen (mittlerer Durchmesser) liegen zwischen 50 und 3.000 µm. Die Eigenschaften der superabsorbierenden Polymerisate, z. B. ihr Quellungsindex, ihr Wasserrückhaltevermögen und ihre Gelfestigkeit, sind auch von der Teilchengröße abhängig. Je kleiner die Teilchen, desto schneller wird der Gleichgewichtsquellungszustand erreicht.

Durch nachfolgendes Erhitzen der vernetzten superabsorbierenden Polymerisate auf 150 bis 250°C, bevorzugt 170 bis 210°C, für 2 bis 30 Minuten, bevorzugt 5 bis 15 Minuten, lassen sich ihre ausgezeichneten anwendungstechnischen Eigenschaften nochmals deutlich verbessern. Dies betrifft insbesondere die Quellungskinetik, d.h. die Absorptionsgeschwindigkeit für Wasser und andere Flüssigkeiten, und die Gelfestigkeit der gequollenen Polymeren sowie ihr Aufnahmevermögen für wäßrige Flüssigkeiten unter Druck.

Das Quellungsvermögen der erfindungsgemäßen vernetzten superabsorbierenden Materialien beträgt in reinem Wasser bis zu 700 g/g und in physiologischer Kochsalzlösung (0,9%ig) bis 60 g/g.

Die Gelfestigkeiten von mit Wasser oder 0,9 %iger Kochsalzlösung gequollenen superabsorbierenden Pulvern liegen, in Abhängigkeit von ihrem jeweiligen Vernetzungsgrad, zwischen 20 und 100 g (gemessen mit dem Texture Analyzer der Firma Stevens).

Die superabsorbierenden Polymerisate eignen sich vorzüglich zum Einsatz bei der Herstellung von Hygieneartikelen, wie z. B. Babywindeln oder Inkontinenzartikeln, sowie zur Ummantelung von Elektrokabeln. Außerdem können die Produkte in der Landwirtschaft als wasserspeichernde Materialien eingesetzt werden.

### Beispiele

### Beispiel 1

### Herstellung einer vernetzten Polyacrylnitril-Emulsion

In einem mit Rührer, Rückfußkühler, Stickstoffeinleitungsrohr und drei Tropftrichtern ausgerüsteten 2 l-Dreihalskolben werden 49,8 g einer 20,1 gew.-%igen wäßrigen Lösung eines Sulfonsäuregruppen enthaltenden anionischen polymeren Emulgators auf Basis eines alternierenden Copolymerisats aus Maleinsäureanhydrid und Diisobutylen (für Einzelheiten siehe DE-OS 38 07 097, Beispiel 2) und 250 g entionisiertes Wasser vorgelegt. Unter Überleiten von Stickstoff und Rühren wird auf 60°C aufgeheizt und die Lösungen I bis III werden innerhalb von drei Stunden gleichzeitig zudosiert.

| | | |
|---|---|---|
| Lösung I | 198,000 g | Acrylnitril |
| | 2,000 g | Divinylbenzol |
| | | |
| Lösung II | 0,358 g | Wasserstoffperoxid (35%ige wäßrige Lösung) |
| | 100,000 g | entionisiertes Wasser |
| | | |
| Lösung III | 0,203 g | Hydroxymethansulfinsäure, Na-Salz |
| | 100,000 g | entionisiertes Wasser |

Nach Dosierende wird noch 6 Stunden bei 60°C nachgerührt. Danach werden die Restmonomeren durch Vakuumdestillation entfernt. Nach Filtrieren über ein 100 µm Filtertuch erhält man eine feinteilige Emulsion mit einem Feststoffgehalt von 28,4 Gew.-%.
- Mittlerer Teilchendurchmesser:: 165 nm (bestimmt durch Laser Korrelations Spektroskopie)

### Herstellung des superabsorbierenden Polymerisats

In einem 2 l-Vierhalskolben, der mit Rückflußkühler, Thermometer, Tropftrichter und Rührer ausgerüstet ist, werden 186,6 g der nach Beispiel 1 hergestellten vernetzten Polyacrylnitril-Emulsion, 92,4 g entionisiertes Wasser und 200 g einer 10 gew.-%igen Natriumhydroxidlösung unter Rühren vorgelegt. Unter Rühren und Überleiten von Stickstoff wird das Reaktionsgemisch auf 95°C erhitzt. Während des Aufheizens ändert sich die Farbe des Polymerisats von farblos über gelb nach dunkelrot, um schließlich wieder farblos zu werden. Nach Erreichen eines Hydrolysegrades von 20 bis 50 % - ermittelt durch quantitative Bestimmung des austretenden Ammoniaks - wird das erhaltene Reaktionsgemisch auf 30 bis 40°C abgekühlt und die nicht verbrauchte Natronlauge durch langsames Zudosieren von Salzsäure (innerhalb von 30 bis 60 Minuten) neutralisiert.

Durch portionsweises Zugeben von Ethanol bei 25 bis 30°C wird unter starkem Rühren das vernetzte superabsorbierende Polymerisat ausgefällt. Dabei beträgt das Volumenverhältnis von Ethanol zu Wasser 1:1. Nach Abfiltrieren und Trocknen im Vakuumtrockenschrank bei 60°C erhält man ca. 90 g eines farblosen Pulvers, das nach dem Zerkleinern in einem Mixer aus Teilchen eines Durchmessers von 200 bis 3.000 µm besteht. Es kann durch Siebung klassiert werden.

### Thermische Behandlung des vernetzten superabsorbierenden Polymerisats

Das getrocknete superabsorbierende Polymerisat wird in einem Trockenschrank ca. 15 Minuten bei 180°C gehalten.

### Messung des Quellungsgrades

Es werden ca. 200 mg des superabsorbierenden Polymerisats in ein 300 ml-Becherglas eingewogen und mit 200 ml destilliertem Wasser bzw. 30 ml einer 0,9 %igen Natriumchloridlösung übergossen und bei 20°C stehengelassen. Nach Erreichen des Gleichgewichtsquellungsgrades wird das erhaltene Gel über ein Filtertuch mit der Maschenweite 50 µm abfiltriert und ausgewogen. Der Quellungsgrad berechnet sich dann aus dem Verhältnis Auswaage:Einwaage in g/g. Jede einzelne Bestimmung wird dreimal durchgeführt. Die Messgenauigkeit ist ± 5 %.

Quellungsgrad in Wasser ohne thermische Nachbehandlung: 370 g/g, nach thermischer Nachbehandlung: 290 g/g.

Quellungsgrad in 0,9%iger Natriumchloridlösung ohne thermische Nachbehandlung: 38,5 g/g, nach thermischer Nachbehandlung: 32,3 g/g.

### Messung der Gelfestigkeit

Apparatur: Stevens L.F.R.A. Texture Analyzer
Messprinzip: Eine zylindrische oder konische Prüfsonde wird mit einer vorgewählten Geschwindigkeit und Strecke in das Gelprüfmuster gedrückt. Die daraus resultierende Kraft in Gramm wird gemessen und digital angezeigt.
Durchführung der Messungen: 1 g der zu untersuchenden Probe wird in 170 ml entionisiertem Wasser 1 Stunde lang gequollen. Danach wird das Gel in ein 150 ml-Becherglas überführt und mit einem Spatel bis zur Homogenität gerührt. Nach Temperierung der Probe bei 20°C wird das Messgerät auf eine Geschwindigkeit von 1 mm/sec und einen Penetrationsweg von 10 mm sowie auf das Prüfprogramm "normal" eingestellt. Als Prüfsonde verwendet man die Zylinderprüfsonde mit der Bezeichnung TA 3, welche einen Durchmesser von 1 inch aufweist. Von jeder Probe werden mindestens zwei Messungen durchgeführt.

### Beispiele 2 bis 8

Die Ergebnisse der Beispiele 2 bis 8 sind in der Tabelle 1 zusammengefaßt. Dabei wurden PAN-Emulsionen mit ansteigendem Vernetzungsgrad eingesetzt. Die nach der Hydrolyse erhaltenen superabsorbierenden Polymeren wurden mittels Siebung nach drei Größenklassen getrennt: <200 µm, 200-1.000 µm und 1.000-3.000 µm.

In den beiden letzten Spalten der Tabelle sind die Quellungsgrade der Produkte in destilliertem Wasser und in 0,9%iger NaCl-Lösung aufgeführt.
Wie aus dieser Versuchsreihe deutlich zu erkennen ist, nimmt das Wasserabsorptionsvermögen der Proben mit zunehmendem Vernetzungsgrad sowohl in destilliertem Wasser als auch in 0,9%iger NaCl-Lösung ab, während die Gelfestigkeit zunimmt (s. Tabelle 2).

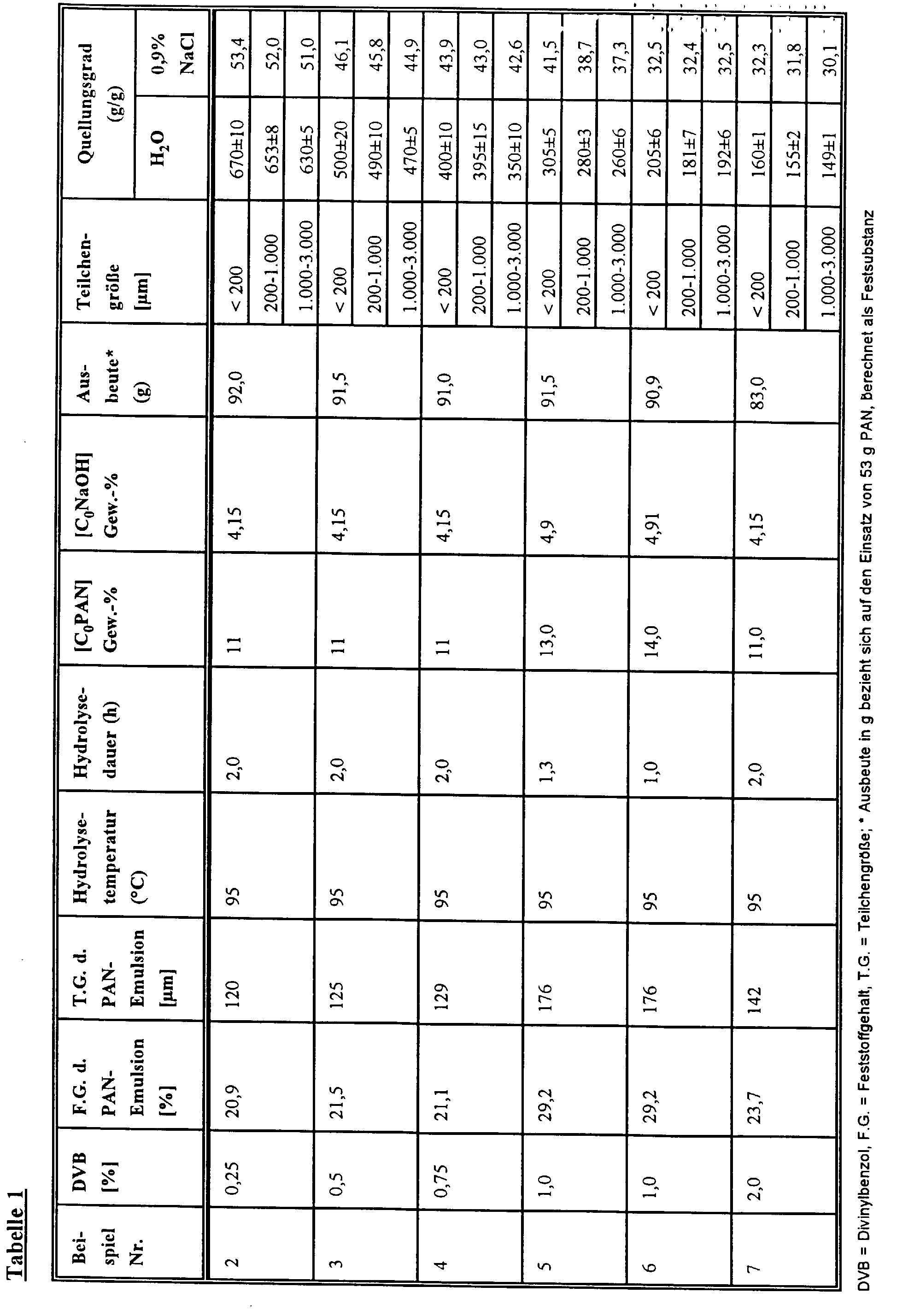

Tabelle 2 zeigt die Ergebnisse der mit dem Stevens Texture Analyzer an einigen ausgewählten Beispielen aus Tabelle 1 (Beispiele 2 bis 4) durchgeführten Messungen der Gelstärke.

**Tabelle 2**

| **Beispiel Nr.** | **[%] DVB im Polymeren** | **Gelfestigkeit (g)** | | |
|---|---|---|---|---|
| | | **vor thermischer Behandlung** | **nach thermischer Behandlung** | |
| | | | **180°C** | **200°C** |
| 2 | 0,25 | 30 | 54 | 82 |
| 3 | 0,50 | 47 | 73 | ---- |
| 4 | 0,75 | 66 | 81 | 99 |

Aus dieser Tabelle 2 ist deutlich zu erkennen, wie mit zunehmendem Vernetzungsgrad die Gelfestigkeit der superabsorbierenden Polymerisate zunimmt. Außerdem wird deutlich, daß durch eine kurzzeitige thermische Nachbehandlung bei 180°C bzw. 200°C der Produkte die Gelfestigkeit noch weiter erhöht werden kann, wobei Werte bis zu 99 g erreicht werden (s. Beispiel 4).

## Patentansprüche

1. Superabsorbierende Polymerisate auf Basis teilhydrolysierter, durch den Einbau polyfunktioneller Monomerer vernetzter Homo- und/oder Copolymerisate des Acrylnitrils, worin 30 bis 80 Mol-% der Nitrilgruppen in Carboxylatgruppen, 20 bis 70 Mol-% der Nitrilgruppen in Carbonamidgruppen und 0 bis 20 Mol-% der Nitrilgruppen unverändert erhalten sind, und die vernetzten Polymerisate ein Quellungsvermögen von bis zu 700 g/g in Wasser und bis zu 60 g/g in physiologischer Kochsalzlösung sowie eine Gelfestigkeit von 20 g bis 100 g besitzen.

2. Superabsorbierende Polymerisate nach Anspruch 1, in denen 30 bis 50 Mol-% der Nitrilgruppen in Carboxylatgruppen und 20 bis 50 Mol-% in Carbonamidgruppen umgewandelt sind, während 5 bis 20 Mol-% der Nitrilgruppen unverändert vorliegen.

3. Verfahren zur Herstellung von superabsorbierenden Polymerisaten, dadurch gekennzeichnet, daß man feinteilige wäßrige Emulsionen von vernetzten Homo- und/oder Copolymerisaten des Acrylnitrils durch Umsetzung mit wäßrigen Lösungen von Alkalihydroxiden partiell hydrolysiert, die Polymerisate durch Zugabe von mit Wasser mischbaren organischen Lösungsmitteln als Pulver ausfällt, abtrennt, trocknet und dann gegebenenfalls für kurze Zeit erhitzt.

4. Verfahren nach Anspruch 3, worin zur Herstellung der vernetzten Homound/oder Copolymerisate des Acrylnitrils polyfunktionelle Monomere in Mengen zwischen 0,2 und 4,0 Gew.-%, bezogen auf die eingesetzten monofunktionellen Monomeren, verwendet werden.

5. Verfahren nach Anspruch 3, worin Divinylbenzol als polyfunktionelles Monomeres zur Herstellung der feinteiligen wäßrigen Emulsionen von vernetzten Homo- und/oder Copolymerisaten des Acrylntrils verwendet wird.

6. Verfahren nach Anspruch 3, worin Divinylbenzol in Mengen zwischen 0,5 und 1,0 Gew.-%, bezogen auf die eingesetzten monofunktionellen Monomeren, verwendet wird.

7. Verfahren nach Anspruch 3, worin das molare Verhältnis der Nitrilgruppen der Polymerisate zu den Alkalihydroxiden 1:1 bis 1:0,1 ist.

8. Verfahren nach Anspruch 3, worin die Umsetzung bei 50 bis 100°C durchgeführt wird.

9. Verfahren nach Anspruch 3, worin 20 bis 90 Mol-% der Nitrilgruppen des vernetzten Acrylnitrilpolymerisats in Carboxylat- bzw. Amidgruppen umgewandelt werden.

10. Verfahren nach Anspruch 3, worin zur Ausfällung der Pulver mit Wasser mischbare organische Lösungsmittel eingesetzt werden.

11. Verfahren nach Anspruch 3, worin die ausgefällten Pulver 2 bis 30 Minuten auf 150 bis 250°C erhitzt werden.

12. Verwendung der superabsorbierenden Polymerisate gemäß Anspruch 1 in Sanitärartikeln, wie z. B. Windeln, als wasserspeichernde Materialien in der Landwirtschaft oder bei der Ummantelung von Elektrokabeln.

## Claims

1. Superabsorbent polymers based on partly hydrolysed homopolymers and/or copolymers of acrylonitrile which are cross-linked through the incorporation of polyfunctional monomers, in which polymers from 30 to 80 mol-% of the nitrile groups are converted into carboxylate groups, from 20 to 70 mol-% of the nitrile groups are converted into carbonamide groups and 0 mol-% to 20 mol-% of the nitrile groups are unchanged, and the said cross-linked polymers possess a swelling capacity of up to 700 g/g in water and of up to 60 g/g in physiological saline and a gel strength of from 20 g to 100 g.

2. Superabsorbent polymers according to claim 1, wherein from 30 to 50 mol-% of the nitrile groups are converted into carboxylate groups and from 20 to 50 mol-% are converted into carbonamide groups, while 5 to 20 mol-% are unchanged.

3. Method for the preparation of superabsorbent polymers, characterised in that fine-particled aqueous emulsions of cross-linked homopolymers and/or copolymers of acrylonitrile are partly hydrolysed by reaction with aqueous solutions of alkali metal hydroxides, the polymers are precipitated as powders by addition of organic solvents which are miscible with water, are separated out, dried and then optionally heated for a short period.

4. Method according to claim 3, wherein, in order to prepare the cross-linked homopolymers and/or copolymers of acrylonitrile, polyfunctional monomers are used in quantities of between 0.2 and 4.0% by weight, referred to the monofunctional monomers employed.

5. Method according to claim 3, wherein divinylbenzene is used as a polyfunctional monomer for the preparation of fine-particled aqueous emulsions of cross-linked homopolymers and/or copolymers of acrylonitrile.

6. Method according to claim 3, wherein divinylbenzene is used in quantities of between 0.5 and 1.0% by weight, referred to the monofunctional monomers employed.

7. Method according to claim 3, wherein the molar ratio of the nitrile groups of the polymer to the alkali metal hydroxides is from 1:1 to 1:0.1.

8. Method according to claim 3, wherein the reaction is carried out at from 50 to 100°C.

9. Method according to claim 3, wherein from 20 to 90 mol-% of the nitrile groups of the cross-linked acrylonitrile polymer are converted into carboxylate groups or amide groups.

10. Method according to claim 3, wherein organic solvents which are miscible with water are employed to precipitate the powders.

11. Method according to claim 3, wherein the precipitated powders are heated to 150 to 250°C for 2 to 30 minutes.

12. Use of the superabsorbent polymers according to claim 1 as sanitary articles such as, for example, nappies, as materials for water storage in agriculture or in the sheathing of electric cables.

## Revendications

1. Polymères superabsorbants à base d'homopolymères et/ou copolymères d'acrylonitrile réticulés par l'incorporation de monomères polyfonctionnels, partiellement hydrolysés, où 30 à 80 % molaires des groupes nitriles sont transformés en groupes carboxylates, 20 à 70 % molaires des groupes nitriles sont transformés en groupes carbonamides et 0 à 20 % molaires des groupes nitriles ne sont pas modifiés, et les polymères réticulés possèdent un pouvoir de gonflement pouvant atteindre 700 g/g dans l'eau et jusqu'à 60 g/g dans la solution physiologique de chlorure de sodium ainsi qu'une résistance de gel de 20 g à 100 g.

2. Polymères superabsorbants selon la revendication 1, où 30 à 50 % molaires des groupes nitriles sont transformés en groupes carboxylates et 20 à 50 % molaires sont transformés en groupes carbonamides, tandis que 5 à 20 % molaires des groupes nitriles sont non modifiés.

3. Procédé de préparation de polymères superabsorbants, caractérisé en ce qu'on hydrolyse partiellement des émulsions aqueuses fines d'homopolymères et/ou de copolymères réticulés de l'acrylonitrile par réaction avec des solutions aqueuses d'hydroxyde d'alcalin, on précipite les polymères par addition de solvants organiques miscibles à l'eau, on sépare, on sèche et ensuite le cas échéant on chauffe pendant un court instant.

4. Procédé selon la revendication 3, où pour préparer les homo- et/ou copolymères réticulés de l'acrylonitrile on utilise des monomères polyfonctionnels en des quantités comprises entre 0,2 et 4,0 % en poids par rapport aux monomères monofonctionnels utilisés.

5. Procédé selon la revendication 3, où on utilise le divinylbenzène comme monomère polyfonctionnel pour préparer les émulsions aqueuses fines des homo- et/ou copolymères réticulés de l'acrylonitrile.

6. Procédé selon la revendication 3, où on utilise le divinylbenzène en des quantités comprises entre 0,5 et 1,0 % en poids par rapport aux monomères monofonctionnels mis en oeuvre.

7. Procédé selon la revendication 3, où le rapport molaire des groupes nitriles des polymères aux hydroxydes d'alcalin est 1:1 à 1:0,1.

8. Procédé selon la revendication 3, où on réalise la réaction entre 50 et 100°C.

9. Procédé selon la revendication 3, où on transforme 20 à 90 % molaires des groupes nitriles des polymères acrylonitriles réticulés en groupes carboxylates ou amides.

10. Procédé selon la revendication 3, où pour précipiter la poudre on utilise des solvants organiques miscibles à l'eau.

11. Procédé selon la revendication 3, où on chauffe entre 150 et 250°C la poudre précipitée pendant 2 à 30 min.

12. Utilisation des polymères superabsorbants selon la revendication 1 dans des articles sanitaires, comme par exemple des couches, comme matériaux absorbant l'eau dans l'agriculture ou pour l'enrobage de câbles électriques.
